# EUROPEAN PATENT APPLICATION

(11) **EP 2 034 312 A1**
(43) Date of publication of application: **11.03.2009**
(21) Application number: 07767287.1
(22) Date of filing: 20.06.2007
(51) Int. Cl.: G01N 33/53, C12M 1/00, C12Q 1/68, G01N 33/543, G01N 33/566, C12N 15/09

(54) **METHOD OF DETECTING TARGET NUCLEIC ACID AND CONTAINER USED IN THE DETECTION METHOD**

(30) Priority: 22.06.2006 JP 2006172522
(71) Applicant: Olympus Corporation, Tokyo 151-0072 (JP)
(72) Inventor: TANIGAMI, Yasuo, Tokyo 151-0072 (JP); HASHIDO, Kento, Tokyo 151-0072 (JP)
(74) Representative: von Hellfeld, Axel
(86) International application number: PCT/JP2007/062449
(87) International publication number: WO 2007/148733

(57) **Abstract**

An object is to provide a method of easily detecting a target nucleic acid with high precision without using a hybridization method and a container used for the detecting method. The method of detecting the target nucleic acid 30, in which a first ligand 31 and a second ligand 32, which are ligands of different kinds, are bonded to a nucleic acid of a detection object, includes steps of preparing a first particle 33 bonded with a plurality of first receptors 331 to which the first ligand 31 specifically binds and a second particle 34 bonded with a plurality of second receptors 341 to which the second ligand 32 specifically binds, mixing a sample which may have the target nucleic acid 30, the first particle 33 and the second particle 34 in a container having a precipitation capturing part on a bottom thereof, and observing a capturing distribution of agglomerates including the target nucleic acid 30 with the first particle 33 and the second particle 34 where the particles are linked through the bonding between the first ligand and the first receptor and the bonding between the second ligand and the second receptor, respectively, and/or a sediment of the first particle 33 and the second particle 34, on the bottom of the container, after the mixing step.

[Sequence Listing]

<110>Olympus Corporation
<120>Method for detecting target nucleic acid and container for its
detection
<130>07P01685
<160>9
<210>1
<211>8
<212>PRT
<213> Artificial Sequence
<220>
<223>Designed peptide to act as a ligand in target nucleic acid.
<400>1
Asp Tyr Lys Asp Asp Asp Asp Lys
1               5

<210>2
<211>18
<212>DNA
<213>Artificial Sequence
<220>
<223>Designed DNA fragment to act as a primer in PCR method for amplification
of target sequence SEQ ID NO:5.
<400>2
ctgtctgacg agatagca 18

<210>3
<211>18
<212>DNA
<213>Artificial Sequence
<220>
<223>Designed DNA fragment to act as a primer in PCR method for amplification
of target sequence SEQ ID NO:5.
<400>3
ctgtctgacg agatagcg 18

<210>4
<211>38
<212>DNA
<213>Artificial Sequence
<220>
<223>Designed DNA fragment to act as a primer in PCR method for amplification
of target sequence SEQ ID NO:5.
<400>4
acactagtaaa tcacagcact cttacaggtc atgagaa 38

<210>5
<211>87
<212>DNA
<213>human
<400>5
ctgtctgacg agatagcrca ggtcaggtgg gctcactcta agcccctgat tctcatgacc 60
tgtaagagtg ctgtgattta ctagtgt 87

<210>6
<211>87

<212>DNA
<213>Artificial Sequence
<220>
<223>PCR product using primer SEQ ID NO:2 and template SEQ ID NO:5
<400>6
ctgtctgacg agatagcaca ggtcaggtgg gctcactcta agcccctgat tctcatgacc 60
tgtaagagtg ctgtgattta ctagtgt 87

<210>7
<211>87
<212>DNA
<213>Artificial Sequence
<220>
<223>PCR product using primer SEQ ID NO:4 and template SEQ ID NO:5
<400>7
acactagtaa atcacagcac tcttacaggt catgagaatc aggggcttag agtgagccca 60
cctgacctgt gctatctcgt cagacag 87

<210>8
<211>87
<212>DNA
<213>Artificial Sequence
<220>
<223>PCR product using primer SEQ ID NO:3 and template SEQ ID NO:5
<400>8
ctgtctgacg agatagcgca ggtcaggtgg gctcactcta agcccctgat tctcatgacc 60
tgtaagagtg ctgtgattta ctagtgt 87

<210>9
<211>87
<212>DNA
<213>Artificial Sequence
<220>
<223>PCR product using primer SEQ ID NO:4 and template SEQ ID NO:5
<400>9
acactagtaa atcacagcac tcttacaggt catgagaatc aggggcttag agtgagccca 60
cctgacctgc gctatctcgt cagacag 87

[Sequence Listing]

<110>Olympus Corporation
<120>Method for detecting target nucleic acid and container for its
detection
<130>07P01685
<160>9
<210>1
<211>8
<212>PRT
<213> Artificial Sequence
<220>
<223>Designed peptide to act as a ligand in target nucleic acid.
<400>1
Asp Tyr Lys Asp Asp Asp Asp Lys
 1               5

<210>2
<211>18
<212>DNA
<213>Artificial Sequence
<220>
<223>Designed DNA fragment to act as a primer in PCR method for
amplification of target sequence SEQ ID NO:5.
<400>2
ctgtctgacg agatagca 18

<210>3
<211>18
<212>DNA
<213>Artificial Sequence
<220>
<223>Designed DNA fragment to act as a primer in PCR method for
amplification of target sequence SEQ ID NO:5.
<400>3
ctgtctgacg agatagcg 18

<210>4
<211>38
<212>DNA
<213>Artificial Sequence
<220>
<223>Designed DNA fragment to act as a primer in PCR method for
amplification of target sequence SEQ ID NO:5.
<400>4
acactagtaaa tcacagcact cttacaggtc atgagaa 38

<210>5
<211>87
<212>DNA
<213>human
<400>5
ctgtctgacg agatagcrca ggtcaggtgg gctcactcta agcccctgat tctcatgacc 60
tgtaagagtg ctgtgattta ctagtgt 87

<210>6
<211>87
<212>DNA
<213>Artificial Sequence
<220>
<223>PCR product using primer SEQ ID NO:2 and template SEQ ID NO:5
<400>6
ctgtctgacg agatagcaca ggtcaggtgg gctcactcta agcccctgat tctcatgacc 60
tgtaagagtg ctgtgattta ctagtgt 87

<210>7
<211>87
<212>DNA
<213>Artificial Sequence
<220>
<223>PCR product using primer SEQ ID NO:4 and template SEQ ID NO:5
<400>7
acactagtaa atcacagcac tcttacaggt catgagaatc aggggcttag agtgagccca 60
cctgacctgt gctatctcgt cagacag 87

<210>8
<211>87
<212>DNA
<213>Artificial Sequence
<220>
<223>PCR product using primer SEQ ID NO:3 and template SEQ ID NO:5
<400>8
ctgtctgacg agatagcgca ggtcaggtgg gctcactcta agcccctgat tctcatgacc 60
tgtaagagtg ctgtgattta ctagtgt 87

<210>9
<211>87
<212>DNA
<213>Artificial Sequence
<220>
<223>PCR product using primer SEQ ID NO:4 and template SEQ ID NO:5
<400>9
acactagtaa atcacagcac tcttacaggt catgagaatc aggggcttag agtgagccca 60
cctgacctgc gctatctcgt cagacag 87

## Description

### Technical Field

The present invention relates to a method of detecting presence/absence of a nucleic acid of a detection object and a concentration thereof, and a container used for the detecting method.

### Background Art

As the representative technology of detecting a specific target nucleic acid present in a sample, a hybridization method has been known (see Patent Document 1). This method is a technology that allows detecting an extremely small amount of target nucleic acid of DNAs or RNAs, from a sample including various kinds of nucleic acids, using a probe.

In addition, a method of carrying out detection by agglomerating a specific target molecule present in a sample has been known. As the container for detecting agglomerates which can be obtained by carrying out an agglomeration reaction according to this method, various containers have been suggested up to now. For example, a container which allows determining an agglomeration pattern of the agglomerate with eyes has been suggested (see Patent Document 2).
[Patent Document 1] Japanese Unexamined Patent Application Publication No. 7-51099
[Patent Document 2] Japanese Patent Application Publication No. 63-60854

### Disclosure of the Invention

### Problems that the Invention is to Solve

However, in a real sense, there is a problem in nucleic acids that there may be a non-specific hybridization. For example, if the target nucleic acid has a base sequence similar to the non-target nucleic acid, it is difficult to selectively detect only a target nucleic acid from a sample. Therefore, in the method described in Patent Document 1, there has been a problem that the target nucleic acid may not be necessarily detected with high precision.
The container described in Patent Document 2 is used for the detection of a red blood cell. Since the nucleic acid and the red blood cell are different from each other in the size or the structure of the molecule, there has been a problem that this container cannot be directly used for the detection of the target nucleic acid.

The present invention is contrived to solve the above-described problems. An object of the present invention is to provide a method of easily detecting a target nucleic acid with high precision and a container used for the detecting method without employing hybridization.

### Means for Solving the Problems

In order to solve the above-described problems, the present invention includes the following constitutions.
(1) A method of detecting a target nucleic acid, in which a first ligand and a second ligand, which are ligands of different kinds, are bonded to a nucleic acid of a detection object, the method comprising the steps of: preparing a first particle bonded with a plurality of first receptors to which the first ligand specifically binds and a second particle bonded with a plurality of second receptors to which the second ligand specifically binds; mixing a sample which may have the target nucleic acid, the first particle and the second particle in a container having a precipitation capturing part on a bottom thereof; and observing a capturing distribution of agglomerates including the target nucleic acid with the first particle and the second particle where the particles are linked through the bonding between the first ligand and the first receptor and the bonding between the second ligand and the second receptor, respectively, and/or a precipitation of the first particle and the second particle, on the bottom of the container, after the mixing step.
(2) A method of detecting a target nucleic acid, in which a first ligand and a second ligand, which are ligands of different kinds, are bonded to a nucleic acid of a detection object, the method comprising the steps of: preparing a container in which a plurality of first receptors to which a first ligand specifically binds is bonded to a bottom thereof; preparing a magnetic particle in which a plurality of second receptors to which a second ligand specifically binds is bonded; adding a sample which may have the target nucleic acid and the magnetic particle into the container; and applying a magnetic force from the outside of the container to the magnetic particle and observing a magnetic particle distribution on the bottom of the container, after the adding step.
(3) A method of detecting a target nucleic acid, the method including performing the detecting method according to (1) and the detecting method according to (2).
(4) The method according to any one of (1) to (3), wherein an observation result obtained when a sample (1) including a target nucleic acid having the known concentration is used, an observation result obtained when a sample (2) including no target nucleic acid is used, and an observation result obtained when a sample (3) which may have the target nucleic acid is used are compared so as to check the presence/absence or the concentration of the target nucleic acid in the sample (3).
(5) The method according to any one of (1) to (3), wherein an observation result obtained when a sample including a target nucleic acids having the known different concentrations are used and an observation result obtained when a sample (4) which may have the target nucleic acid is used are compared so as to check the presence/absence or the concentration of the target nucleic acid in the sample (4).
(6) The method according to any one of (1) to (5), wherein the ligand is selected from the group consisting of a hydrophilic organic compound, digoxigenin, fluorescein, alexa, fluorescein isothiocyanate (FITC), 2,4-dinitrophenol (DNP), tetramethyl rhodamine (TAMRA), a polypeptide of 6 or more amino acid residues, a sugar chain having two or more monosaccharides, a biotin, a protein, a polyhistidine, HA, a glutathione S-transferase (GST), a peptide (Flag) including an amino acid sequence represented by SEQ ID No. 1, and derivatives of any of these.
(7) The method according to any one of (1) to (6), wherein the target nucleic acid is prepared by a polymerase chain reaction (PCR) method using a first primer to which the first ligand is bonded and a second primer to which the second ligand is bonded.
(8) The method according to any one of (1) to (6), wherein the target nucleic acid is prepared by a polymerase chain reaction (PCR) method while at least one nucleotide to which the first ligand is bonded is introduced using a first primer; and while at least one nucleotide to which the second ligand is bonded is introduced using the second primer.
(9) The method according to any one of (1) to (8), wherein plural kinds of different target nucleic acids are used, where at least one of the first ligand and the second ligand in the target nucleic acids is a different kind for each of the target nucleic acids.
(10) The method according to any one of (1) to (9), wherein the target nucleic acid is derived from an organism.
(11) The method according to any one of (1) and (3) to (10), wherein the first and second particles have a diameter of 0.1 to 10 µm and are optically distinguishable from each other.
(12) The method according to any one of (1) to (11), wherein a plurality of containers is provided on the same plate and the capacity of each of the container is 0.1 to 0.5 ml.
(13) The method according to any one of (1) and (3) to (12), wherein, the precipitation capturing part of the container is formed on the bottom of thereof having a conical shape, so that a plurality of peak-valley forms consisting of peak portions and valley portions which are alternatingly arranged, and having a diameter-direction cross section of a saw-tooth shape, is concentrically arranged; the length (h) of a first inclined surface connecting a peak and a valley from a center to the outside of the bottom is 0.2 to 20 µm, the length (1) of a second inclined surface connecting a valley and a peak from the center to the outside of the bottom is 0.5 to 50 µm, and an angle (θ₁) between a straight line connected between the peak and the center, and a horizontal plane is 15 to 45°.
(14) The method according to any one of (1) and (3) to (12), wherein, in the precipitation capturing part of the container, a plurality of valley parts is independently provided on the bottom having a conical shape and an angle (θ₂) between the bottom and a horizontal plane is 15 to 45°.
(15) The method according to (14), wherein the diameter (L) of an opening of each of the valley parts is 0.05 to 100 µm and the depth (d) thereof is 0.03 to 60 µm.
(16) A method of detecting a target nucleic acid, the method including performing the detecting method according to (13) and the detecting method according to (14) or (15).
(17) The method according to any one of (2) to (10) and (12), wherein the diameter of the magnetic particle is 0.1 to 20 µm.
(18) The method according to any one of (2) to (10), (12) and (17), wherein the bottom of the container has a substantially semi-spherical shape or a substantially conical surface shape and an angle (θ₃) between the bottom and a horizontal plane is 15 to 45°.

(19) A container used for the method of detecting the target nucleic acid according to any one of (1) and (3) to (12), wherein, the precipitation capturing part of the container is formed on the bottom of thereof having a conical shape, so that a plurality of peak-valley forms consisting of peak portions and valley portions which are alternatingly arranged, and having a diameter-direction cross section of a saw-tooth shape, is concentrically arranged; the length (h) of a first inclined surface connecting a peak and a valley from a center to the outside of the bottom is 0.2 to 20 µm, the length (1) of a second inclined surface connecting a valley and a peak from the center to the outside of the bottom is 0.5 to 50 µm, and an angle (θ₁) between a straight line connected between the peak and the center and a horizontal plane is 15 to 45°.
(20) A container used for the method of detecting the target nucleic acid according to any one of (1) and (3) to (12), wherein a plurality of valley parts is independently provided on the bottom having a conical shape and an angle (θ₂) between the bottom and a horizontal plane is 15 to 45°.
(21) The container according to (20), wherein the diameter (L) of an opening of each of the valley parts is 0.05 to 100 µm and the depth (d) thereof is 0.03 to 60 µm.
(22) A container used for the method of detecting the target nucleic acid according to any one of (2) to (10), (12) and (17), wherein the bottom thereof has a substantially semi-spherical shape or a substantially conical surface shape and an angle (θ₃) between the bottom and a horizontal plane is 15 to 45°.
(23) A container used for detecting a target nucleic acid, in which the container according to any one of (19) to (22) is plurally provided on the same plate.

### Effect of the Invention

According to the present invention, it is possible to easily detect the presence/absence or the concentration of the target nucleic acid by visually observing a sediment capturing distribution on the bottom of a container.

### Brief Description of the Drawings

FIG. 1A is a conceptual diagram showing a method of obtaining a target nucleic acid.
FIG. 1B is a conceptual diagram showing a method of obtaining a target nucleic acid.
FIG. 1C is a conceptual diagram showing a method of obtaining a target nucleic acid.
FIG. 1D is a conceptual diagram showing a method of obtaining a target nucleic acid.
FIG. 2A is a view showing a particle bonded with a receptor.
FIG. 2B is a view showing a particle bonded with a receptor.
FIG. 3 is a conceptual diagram showing a particle when being bonded with a target nucleic acid so as to be agglomerated.
FIG. 4 is a cross-sectional view showing an example of a container according to the present invention.
FIG. 5 is a cross-sectional view showing another example of a container according to the present invention.
FIG. 6 is a view showing an example of a sediment capturing distribution when the container shown in FIG 4 is used.
FIG. 7 is a view showing an example of a sediment capturing distribution when the container shown in FIG 5 is used.
FIG. 8 is a cross-sectional view showing another example of a container according to the present invention.
FIG. 9 is a view showing a magnetic particle bonded with a receptor.
FIG. 10A is a conceptual diagram showing a state in which the container shown in FIG. 8 and the magnetic particle shown in FIG. 9 are connected via a target nucleic acid.
FIG. 10B is a conceptual diagram showing a state in which the container shown in FIG. 8 and the magnetic particle shown in FIG. 9 are connected via a target nucleic acid.
FIG. 10C is a conceptual diagram showing a state in which the container shown in FIG. 8 and the magnetic particle shown in FIG. 9 are connected via a target nucleic acid.
FIG. 11 is a view showing an example of a distribution pattern of the magnetic particle of FIGS. 10A to 10C.

### Reference Numerals

30: target nucleic acid, 31: first ligand, 32: second ligand, 33: first particle, 331: first receptor, 34: second particle, 341: second receptor

### Best Mode for Carrying out the Invention

Hereinafter, the present invention will be described in detail.
A method of detecting a target nucleic acid according to the present invention largely includes a process of obtaining a target nucleic acid having a structure in which a ligand is bonded to a nucleic acid of a detection object and a process of detecting the target nucleic acid through the ligand. Hereinafter, the processes will be described in detail.

### <Process of Obtaining Target Nucleic Acid>

First, the process of obtaining the target nucleic acid will be described. In the present invention, the nucleic acid of the detection object is not specially limited and includes all nucleic acids or nucleic acid analogues including a cDNA, a genomic DNA, a synthetic DNA, an mRNA, all RNAs, an hnRNA, a synthetic RNA and the like. The nucleic acid may be derived from an organism or artificially synthesized.

As the ligand bonded to the nucleic acid of the detection object, a conventional ligand may be used. For example, preferably, the ligand includes, but not limited to, a hydrophilic organic compound, hapten, digoxigenin, fluorescein, alexa, fluorescein isothiocyanate (FITC), 2,4-dinitrophenol (DNP), tetramethyl rhodamine (TAMRA), a polypeptide of 6 or more amino acid residues, a sugar chain having two or more monosaccharides, a biotin, a protein, a polyhistidine, HA, a glutathione S-transferase (GST), a peptide (Flag) including an amino acid sequence represented by SEQ ID No. 1, a derivative thereof or the like. Biotin, digokisigen, fluorescein, alexa are advantageous from the viewpoints of availability and low cost. In addition, the sugar chain or the peptide or the like is advantageous in that a freedom degree is high in the design of a chemical structure and plural kinds can be easily obtained. The ligand to be used may be properly selected according to samples and the nucleic acids of the detection object.

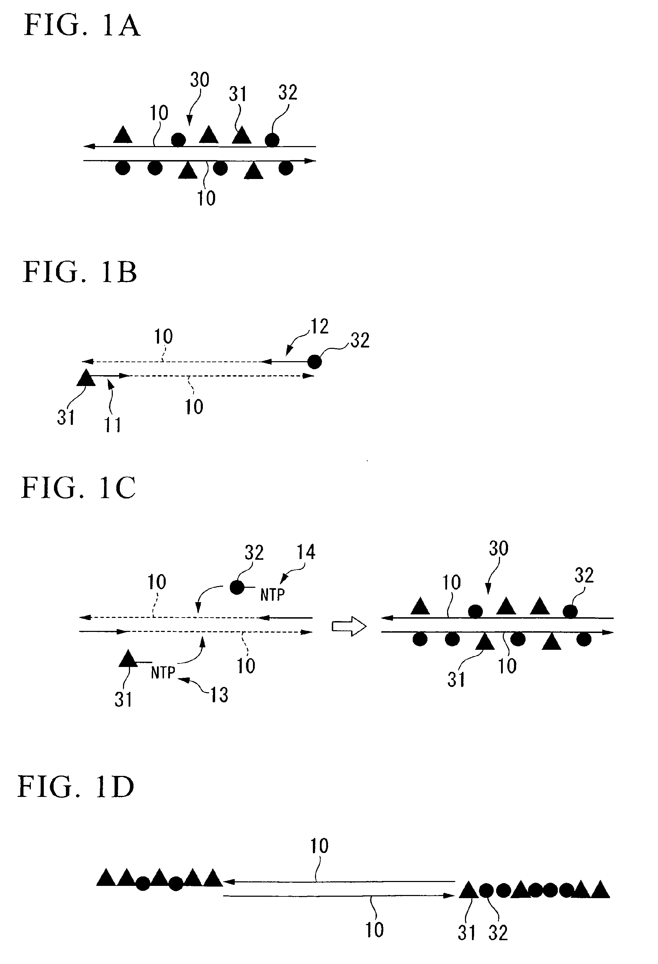

In the detecting method of the present invention, the target nucleic acid bonded with at least one first ligand and at least one second ligand which are ligands of different kind is first formed. The method of obtaining the target nucleic acid having a structure
in which the first and second ligands are bonded to the nucleic acid of the detection object may use, as shown in FIGS. 1A to 1D, various methods including, for example, (i) a method of covalently bonding the first ligand 31 and the second ligand 32 to a base of the nucleic acid 10 and/or 5'- terminal via a linker using light or platinum or via a linker (FIG. 1A) (an example of this method includes a method of covalently bonding a carboxylic group of the ligand and an amino group of a base of the 5'- terminal of the nucleic acid); (ii) a method of a base sequence expansion by a polymerase chain reaction (PCR) using a first primer 11 bonded with the first ligand 31 and a second primer 12 bonded with the second ligand 32 (FIG. 1B) at the time of synthesis of the nucleic acid; (iii) a method of a base sequence expansion while at least one nucleotide 13 bonded with the first ligand 31 and at least one nucleotide 14 bonded with the second ligand 32 are introduced, in PCR (FIG. 1C); and (iv) a method of tailing a nucleotide to 3'- terminal of the nucleic acid which is linked with the first ligand 31 or the second ligand 32, using a terminal dioxydyl transferase (FIG. 1D).
In a sample in which a nucleic acid of a detection object and another nucleic acid of a non-detection object are present together as in a biological sample or any other sample, the methods of (ii) and (iii) are suitable. If the nucleic acid of the detection object is solely present, any of the methods may be used. In addition, for example, the nucleic acid of the detection object included in the biological sample or the like may be detected either after being amplified by the PCR method or without being amplified.

As shown in FIG. 1, the ligand of the target nucleic acid 30 includes two kinds of ligands, that is, a first ligand 31 and a second ligand 32. The structure of the nucleic acid is flexible. In the present invention, as described below, the target nucleic acid is necessarily bonded with different kinds of a first particle and a second particle.
In addition, the number of the first ligands or the second ligands in the target nucleic acid may be one or two or more. Since the volume ratio of the nucleic acid to the particle is significantly large, a possibility that three or more particles are bonded to one nucleic acid is low. Accordingly, although a plurality of ligands is present in one target nucleic acid, it is believed that an agglomeration degree of the particle bonded to the target nucleic acid is not influenced.

When plural kinds of target nucleic acids are present, for example, the methods of (ii) and (iii) may be simultaneously performed so as to simultaneously prepare the plural kinds of target nucleic acids. In this case, preferably, at least one among the first ligand and the second ligand is a different kind for each of the target nucleic acids.

### <Process of Detecting Target Nucleic Acid>

Next, the process of detecting the target nucleic acid will be described. The detecting process is largely divided into two methods. Although it is still possible to carry out a detection with any of the methods alone, a detection result with high precision can be obtained by a combination of the two methods. Hereinafter, the two methods will be sequentially described.

### [First Method] (Process of Preparing First Particle and Second Particle)

A first particle bonded with a plurality of first receptors to which the first ligand specifically binds and a second particle bonded with a plurality of second receptors to which the second ligand specifically binds are prepared.
The particle used in the present invention is a dispersion particle and indicates, for example, a particle dispersed in a solution like a colloid particle. Although a latex particle is suitably used, it is not limited thereto in the present invention. The diameter of the first particle and the second particle is preferably 0.1 to 10.0 nm. If the particle having such a diameter is used, it is easy to obtain a stable agglomerate.

In the present invention, the specific binding refers to bonding based on the force between molecules with high specificity, in which the structure is selectively formed between specific materials such as an antigen and an antibody.

The latex particle is mainly composed of polystyrene and is copolymerized with a methacrylic acid in order to improve hydrophilic and dispersive properties. The latex particle includes a plain type latex particle without a functional group in the surface thereof and a functional group type latex particle with a functional group. The charge of the surface of the plain type latex particle has a negative charge due to the presence of the methacrylic acid and may be ionically bonded to an area having a positive charge in a protein molecule. A hydrophobic bond with the protein molecule is possible. Since the plain type latex particle adsorbs a protein by mixing the protein, the fixing of the protein is easy. Although various receptors may be used as described below, the plain type latex particle is suitably used as the particle if various types of proteins are used.

The latex particle with the functional group is designed such that a carboxylic group or an amino group is exposed on the surface thereof and various types of functional group type latex particle are available. If the receptor is bonded to the functional group type latex particle, a functional group in the functional group type latex particle and the functional group of the receptor may be bonded. Conventional methods, such as an EDAC method of bonding a carboxylic acid and an amino acid using a water-soluble carbodiimide, a method of bonding a carboxylic acid and an amino group by mixing 1-ethyl-3-(3-dimethylaminopropyl) carbodiimide hydrochloride (EDC) and N-hydroxy succinimide (NHS) in advance, a method of cross-linking amino groups using a linker having bipolarity, and a method of bonding an activated aldehyde group or tosyl group to a functional group in the receptor, may be used. In the present invention, although any one of the conventional methods may be used so long as the effect of the present invention is not hindered, the EDAC method is particularly preferable. In addition, if various types of proteins are used as the receptor like the plain type latex particle, the functional group type latex particle is suitably used.
In addition, instead of the latex particle, other particles may bond the receptor by any one of the conventional methods according to the kinds of the particle.

A receptor to which the first ligand in the target nucleic acid specifically binds is used for a first receptor bonded to the first particle and a receptor to which the second ligand specifically binds is used in the second receptor bonded to the second particle.
As the receptor, for example, an antibody, lectin, streptavidin or the like may be used, but it is not limited thereto in the present invention. Among them, a protein and more particularly an antibody is suitably used.

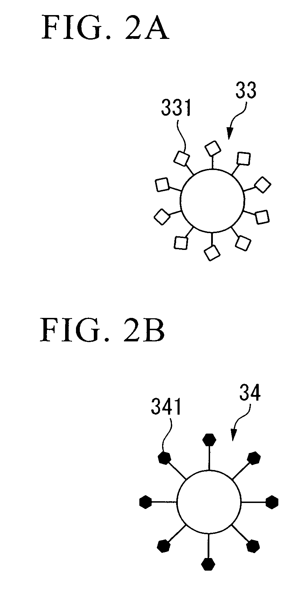

In the present invention, as one embodiment, a single kind of receptor is bonded to one particle. In addition, in the present embodiment, since at least two kinds of ligands are bonded to the target nucleic acid, two or more kinds of particles are prepared with respect to one kind of target nucleic acid. That is, as shown in FIGS. 2A to 2B, a first particle 33 (FIG. 2A) bonded with a first receptor 331 to which the first ligand 31 specifically binds and a second particle 34 (FIG. 2B) bonded with a second receptor 341 to which the second ligand 32 specifically binds are prepared.

As another embodiment of the present invention, two or more kinds of receptors may be bonded to one particle. In the present embodiment, among the two or more kinds of ligands in one target nucleic acid, a receptor to which one kind of ligand specifically binds and a receptor excluding receptors to which the remaining kind of ligands specifically bind are bonded to one particle. That is, only one kind of ligand among the two or more kinds of ligands in the target nucleic acid is bonded to one particle.

It is preferable that the first particle and the second particle are optically distinguishable from each other. In more detail, for example, the first particle and the second particle have different colors instead of the same color. Accordingly, the identification of the particles in a manipulation step becomes facilitated and, even when magnetic agglomeration occurs by one kind of particle in the result of observing an agglomeration image, the distinguishment of the particles becomes facilitated by the color. Thus, a possibility that the magnetic agglomeration is erroneously determined to the agglomeration due to the first particle and the second particle is low. That is, it is possible to detect the target nucleic acid with higher precision.

In addition, although the bonding of the receptor to the particle in the present process may be performed when the target nucleic acid is detected, the particle to which the receptor is bonded in advance may be subjected to the detection. In addition, the present process may be performed before or after the process of preparing the target nucleic acid.

### (Process of Mixing Sample having Possibility of including Target Nucleic Acid, with First Particle and Second Particle)

Next, a process of mixing a sample which may have the target nucleic acid, with the first particle and the second particle will be described.
The present process is performed in the container having a precipitation capturing part on a bottom thereof. If the sample which may have the target nucleic acid that is subjected to the detection actually includes the target nucleic acid, the target nucleic acid and the particle are connected by the bonding between the first ligand and the first receptor and the bonding between the second ligand and the second receptor, and an agglomerate occurs as described below. If the sample subjected to the detection does not include the target nucleic acid, this bonding does not occur and thus the agglomerate does not occur.
The present process may be performed by simultaneously mixing the sample which may have the target nucleic acid, with the first particle and the second particle, or by sequentially mixing the first particle and the second particle to the sample having the possibility of including the target nucleic acid. The sample which may have the target nucleic acid, the first particle and the second particle are preferably used as solutions.

The solution used in the present process is preferably a buffer solution and more preferably a buffer solution enhancing agglomeration. As a preferable buffer solution, for example, a buffer solution including the following composition: 200 mM MOPSO (pH7.4), 4% dextran, 100 mM NaCl and 0.1% sodium azide may be used, but the buffer solution may be properly selected according to the kinds of the target nucleic acid and the particle. If different kinds of buffer solutions are mixed in the present process, it is preferable that the particle is used after being cleaned by the different buffer solution two times or three times.

If the amount of the first and second particles subjected to mixing is too large, detection precision deteriorates. Thus, the amount of the first and second particles is preferably slightly more than that of the target nucleic acid and is more preferably 0.01 to 0.5 w/v% with respect to the target nucleic acid of several tens to several hundreds of nM.

If an antigen and an antibody are, for example, used as a combination of the ligand and the receptor, the present process is preferably performed at 0 to 37 °C for 5 to 30 minutes. Even in the other combination of the ligand and the receptor, a suitable temperature, time and buffer solution may be properly selected according to the kinds of the combination.

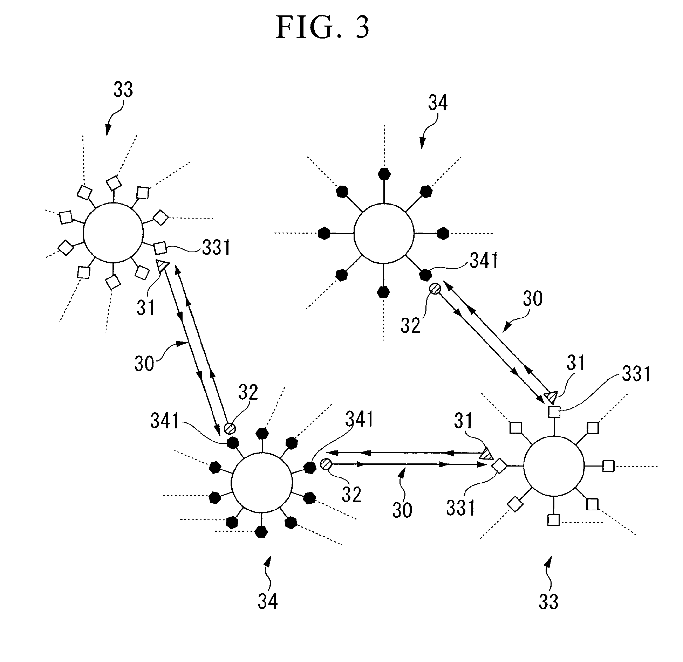

FIG. 3 shows a state in which the target nucleic acid and the first and second particles are bonded. In FIG. 3, the first ligand 31 in the target nucleic acid 30 is bonded to the first particle 33 via the first receptor 331. In addition, the second ligand 32 in the target nucleic acid 30 is bonded to the second particle 34 via the second receptor 341. That is, the first particle 33 and the second particle 34 are bonded to each other by bonding one target nucleic acid 30 to the first particle 33 and the second particle 34. In addition, by bonding a plurality of target nucleic acids 30 to one particle and bonding the target nucleic acid 30 to different particles so as to bond a plurality of particles with one another, agglomeration of the particles occurs. In addition, in FIG. 3, a dotted line extending from the receptor schematically shows the bonded nucleic acids.
Accordingly, if the target nucleic acid is included in the sample, agglomeration of the particles occurs.

### (Process of Observing Precipitation Capturing Distribution in the Bottom of Container)

Next, the sediment capturing distribution in the bottom of the container is observed.
The precipitation as used herein indicates the first and second particles precipitated without being subjected to the bonding with the target nucleic acid or the agglomerate. By the present process, it is possible to detect the target nucleic acid.
The presence/absence and the degree of the agglomerate are determined by naked eyes, image processing or the like. As the image processing, for example, a method of analyzing image data input by a CCD camera may be used.

Next, the detailed method of detecting the target nucleic acid will be described.
A sample (1) including the target nucleic acid having the known concentration and bonded with the above-described ligand is used, the target nucleic acid is mixed with the particle by the above-described method, and the capturing distribution of the agglomerate in the bottom of the container is observed and is set to a positive control.
A sample (2) which does not include the target nucleic acid is mixed with the particle by the above-described method, and the capturing distribution of the precipitate in the bottom of the container is observed and is set to a negative control. A sample (3) which may have target nucleic acid is mixed with the particle by the above-described method, the capturing distribution of the precipitate in the bottom of the container is observed, and the positive control and the negative control are compared so as to check the presence/absence or the concentration of the target nucleic acid in the sample (3). In more detail, if the pattern of the capturing distribution in case of using the sample (3) is equal to or similar to the positive control, the sample (3) is determined to be positive and thus it is confirmed that the sample (3) includes the target nucleic acid. In contrast, if the pattern of the capturing distribution in case of using the sample (3) is equal to or similar to the negative control, the sample (3) is determined to be negative and thus it is confirmed that the sample (3) does not include the target nucleic acid. In contrast, if the pattern of the capturing distribution in case of using the sample (3) is between the positive control and the negative control, the sample (3) is determined to be pseudo positive. In this case, there is a possibility that an extremely small amount of target nucleic acid is included in the sample (3). Accordingly, it is possible to easily check the presence/absence of the target nucleic acid in the sample (3). Similarly, for example, by comparing the capturing amount of the precipitate in case of using the sample (3) with the positive control and the negative control, it is possible to check the amount of target nucleic acid in the sample (3).

By setting the capturing distribution of the agglomerate in the bottom of the container, which is obtained using the sample including the target nucleic acid having the known concentration as a control; and comparing the precipitation capturing distribution when a sample (4) which may have the target nucleic acid with the control, it is possible to check the presence/absence or the concentration of the target nucleic acid in the sample (4), as described above.
In addition, when a difference in the precipitation capturing distribution due to a the presence/absence or difference in the concentration of the target nucleic acid is checked and recorded in advance, it is possible to check the presence/absence or the concentration of the target nucleic acid in the sample by only subjecting the sample which may have the target nucleic acid to the detection without using the control. Accordingly, it is possible to simplify the detecting process.

In addition, in the case where plural kinds of target nucleic acids are included in the sample, for example, it is possible to selectively detect one kind of target nucleic acid, by preparing a different set of first and second ligands according to the kinds of the target nucleic acid and individually subjecting particles respectively bonded with the first and second receptors to which the set of first and second ligands in one kind of target nucleic acid among the plural kinds specifically binds to the mixing. Similarly, it is possible to simultaneously detect two or more kinds of target nucleic acids, by preparing particles bonded with the first and second receptors to which the first and second ligands in the target nucleic acid specifically bind according to the kinds of the target nucleic acid with respect to each target nucleic acid and subjecting these particles to the mixing.
Accordingly, it is possible to solely detect the nucleic acid of the detection object or simultaneously detect nucleic acids from the plurality of the detection objects.

Here, in the first and second ligands in the target nucleic acid, although the different ligands may be used according to the kinds of the target nucleic acid as described above, one of the first and second ligands may be commonly used in the plural kinds of nucleic acids. Accordingly, it is possible to simplify the detecting process.

Although, in the above-described detecting method, two kinds of ligands are used with respect to one kind of target nucleic acid, three or more kinds of ligands may be used. In this case, the particles bonded with a plurality of receptors to which the ligands specifically bind may be prepared according to the kinds of the ligand.

### (Container Used for Detecting Target Nucleic Acid)

Hereinafter, the container of the present invention used for detecting the target nucleic acid will be described in detail.
In the present invention, the container may be solely used or a plurality of containers provided on the same plate may be used. In more detail, for example, a 96-well microplate may be used as the container. The number of wells is not limited to 96. In the case where the plurality of containers is used, the capacity of one container is preferably 0.1 to 0.5 ml.

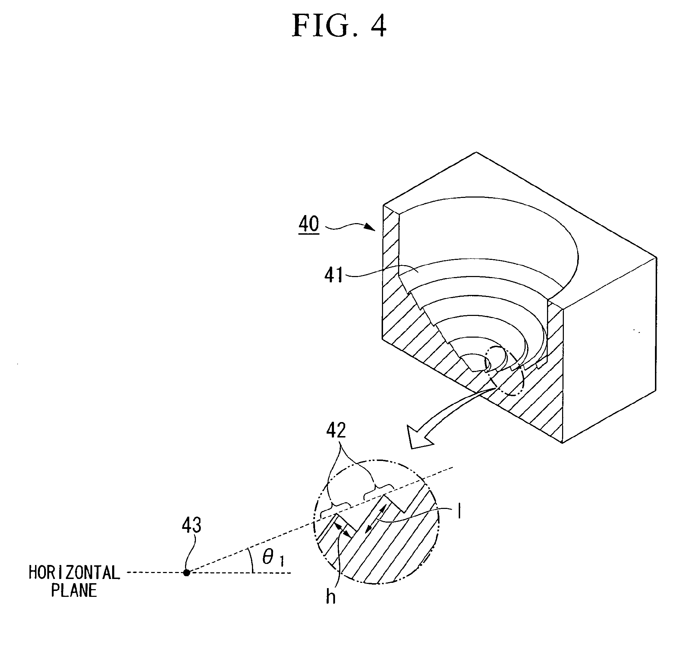

A precipitation capturing part is provided on the bottom of the container of the present invention, and the agglomerate generated by bonding the target nucleic acid and the particle and the sediment other than the agglomerate can be more easily distinguished with high precision. In the detailed description of the precipitation capturing part, for example, a plurality of peak-valley forms 42 having a diameter-direction cross section of a sawtooth shape may be concentrically arranged on a bottom 41 having a conical shape of a container 40 as shown in FIG. 4. With regard to the peak-valley forms 42, the length (h) of a first inclined surface connecting a peak and a valley from the center 43 to the outside of the bottom is preferably 0.2 to 20 µm, the length (1) of a second inclined surface connecting a valley and a peak from the center 43 to the outside of the bottom is preferably 0.5 to 50 µm, and an angle (θ₁) between a straight line connected between the peak and the center 43, and a horizontal plane is preferably 15 to 45°. In addition, the number of peak-valley forms 42 is preferably 10 to 50.

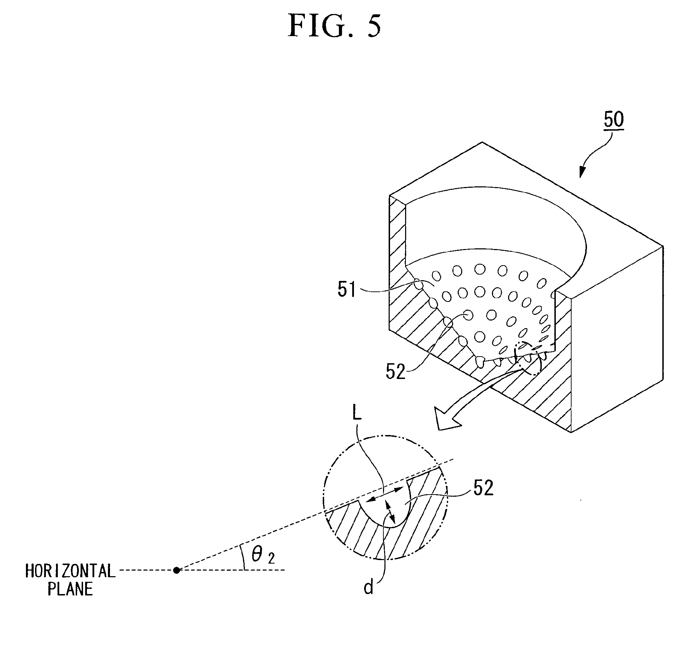

As another detailed example of the precipitation capturing part, for example, a plurality of valley parts 52 may be independently provided on a bottom 51 having a conical shape of a container 50 as shown in FIG. 5. An angle (θ₂) between the bottom 51 and the horizontal plane (outer bottom of the container) is preferably 15 to 45°. In addition, the angle (θ₂) is an angle between the horizontal plane and a straight line connected between the center of the bottom 51 and any one point excluding the valley parts 52 in the inclined surface of the bottom 51.
In addition, the valley part 52 has a substantially semi-spherical shape, the diameter (L) of the opening thereof is preferably 0.05 to 100 µm, and the depth (d) thereof is preferably 0.03 to 60 µm.
The shape of the valley part 52 is not the substantially semi-spherical shape and the valley part 52 may have different shapes and sizes if a plurality, and, more preferably, three to seven first particles and/or second particles can be received. The number of valley parts 52 is several tens to several hundreds.

The size and the shape of the precipitation capturing part are specially found such that the distinguishment between the agglomerate and the sediment other than the agglomerate is easily performed with high precision. By utilizing the container having such a precipitation capturing part, it is possible to easily detect the target nucleic acid with high precision. Accordingly, if the size and the shape of the precipitation capturing part deviate from the above range, it is difficult to distinguish the sediment capturing distribution pattern between the agglomerate and the precipitate other than the agglomerate.
By utilizing the containers described in FIG. 4 and FIG. 5 together, it is possible to detect the target nucleic acid with higher precision.

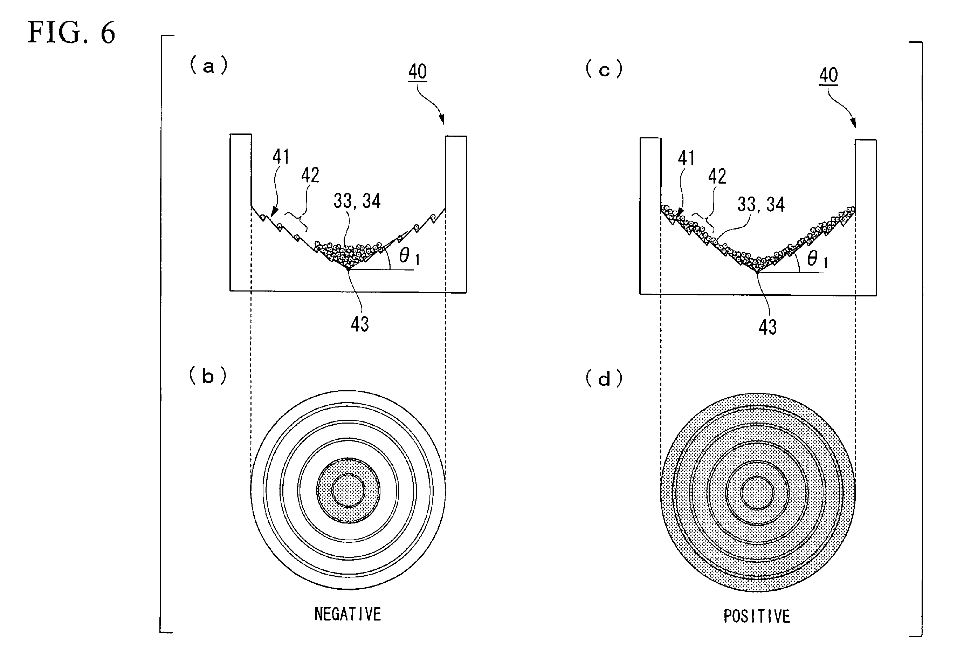

The sediment capturing distribution when the container 40 having the precipitation capturing part shown in FIG. 4 will be described with reference to FIG. 6.
When the target nucleic acid is not included in the sample in which the first particle 33 and the second particle 34 are mixed (negative), as shown in FIG. 6(panel (a) and (b)), the first particle 33 and the second particle 34, which do not participate in the bonding with the target nucleic acid, are precipitated on the bottom 41 so as to mostly climb over the peak-valley form 42 and reach the vicinity of the lowermost part of the bottom. In contrast, when the target nucleic acid is included (positive), as shown in FIG. 6 (panel (c) and (d)), as described above, the first particle 33 and the second particle 34 are bonded with each other via the target nucleic acid and the agglomerate is generated and sedimented. Since the size of the agglomerate is large, the agglomerate stays at the sedimented place of the bottom 41 without climbing over the peak-valley forms 42 so as to be thinly deposited over the entire surface of the bottom 41. If the amount of target nucleic acid is large, the deposition amount of agglomerate is increased and, if the amount of target nucleic acid is small, the deposition amount of agglomerate is decreased. Thus, the color density of the bottom of the container is changed according to the concentration of the target nucleic acid in the sample. The panels (a) and (c) in FIG.6 are longitudinal cross-sectional views of the container 40 in a state in which the agglomerate and/or the sediment other than the agglomerate are captured by the precipitation capturing part and FIG. 6 (panel (b) and (d)) are views when seen from the bottom of the container 40 when viewed from the top.

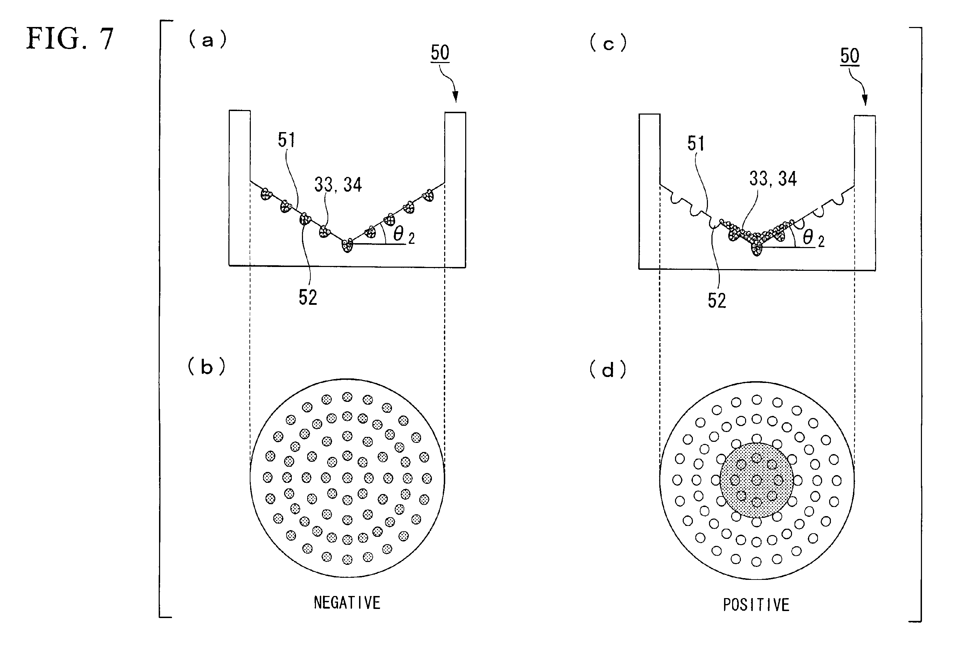

Next, the sediment capturing distribution when the container 50 including the bottom having the shape shown in FIG. 5 is used will be described with reference to FIG. 7.
When the target nucleic acid is not included in the sample in which the first particle 33 and the second particle 34 are mixed (negative), as shown in FIG. 7(panel (a) and (b)), the first particle 33 and the second particle 34, which do not participate in the bonding with the target nucleic acid, are precipitated on the bottom 51 and are mostly captured in the valley part 52 so as not to reach the vicinity of the lowermost part of the bottom 51 such that the particles are held over substantially entire surface of the area, in which the valley part 52 is provided, of the bottom 51. In contrast, when the target nucleic acid is included (positive), as shown in FIG. 7 (panel (c) and (d)), as described above, the first particle 33 and the second particle 34 are bonded with each other via the target nucleic acid and the agglomerate is generated and precipitated. Since the size of the agglomerate is large, the agglomerate mostly reaches the vicinity of the lowermost part of the bottom 51 without being captured by the valley part 52. If the amount of target nucleic acid is large, the deposition amount of agglomerate in the vicinity of the lowermost part of the bottom 51 is increased and the area for covering the bottom 51 is increased and, if the amount of target nucleic acid is small, the deposition amount of agglomerate in the vicinity of the lowermost part of the bottom 51 is decreased and the area for covering the bottom 51 is decreased. Thus, the color density and the area of a color change part of the bottom of the container are changed according to the concentration of the target nucleic acid in the sample. The panels (a) and (c) in FIG. 7 are longitudinal cross-sectional views of the container 50 in a state in which the agglomerate and/or the precipitate other than the agglomerate are captured by the precipitation capturing part and FIGS. 7B and 7D are views the bottom of the container 50 when viewed from the top.

Since the sediment capturing distribution on the bottom of the container varies depending on a difference of the presence/absence or the concentration of the target nucleic acid in the sample subjected to the measurement, it is possible to easily detect the target nucleic acid with high precision.

In addition, for example, the container of the present invention shown in FIG. 4 or 5 may be manufactured by the conventional method such as molding.

### [Second Method] (Bonding of Receptor to Magnetic Particle and Bottom of Container)

Next, a second method of detecting a target nucleic acid will be described. First, a process of bonding, to a magnetic particle, a second receptor to which a second ligand in the target nucleic acid specifically binds will be described.

The magnetic particle to be used includes, for example, a magnetic particle including metal such as triiron tetroxide (Fe₃O₄), diiron trioxide (γ-Fe₂O₃), various types of ferrites, iron, manganese, nickel, cobalt, chrome or the like or an alloy including cobalt, nickel, manganese or the like; a hydrophobic polymer such as polystyrene, polyacrylonitrile, polymethacrylonitrile, polymethylmethacrylate, polycapramide, polyethylene terephthalate, a cross-linked hydrophilic polymer such as polyacrylamide, polymethacrylamide, polyvinylpyrrolidone, polyvinyl alcohol, poly(2-oxyethylacrylate), poly(2-oxyethylmethacrylate), poly(2,3-dioxypropyl acrylate), poly(2, 3-dioxypropyl methacrylate), polyethylene glycol methacrylate or the like in which the magnetic particle is included; a latex such as two to four types of copolymers of a monomer of the polymer, gelatin, liposome, or a particle in which the magnetic particle is fixed on the surface of latex, gelatin and liposome.

The diameter of the magnetic particle is preferably 0.1 µm to 20 µm.
If the diameter deviates from the above range, it is difficult to distinguish the sediment capturing distribution pattern between the agglomerate and the sediment other than the agglomerate.
The method of bonding the second receptor bonded with the second ligand in the target nucleic acid to the magnetic particle includes a method of physically absorbing the receptor or a chemically bonding method.

The physical adsorbing method includes, for example, a method of directly adsorbing and fixing the receptor to the magnetic particle or a method of adsorbing and fixing the receptor after the receptor is chemically bonded to another protein such as albumin.
The chemically bonding method includes, for example, a method of activating and bonding various types of functional groups such as an amino group, a cryboxylic group, a mercapto group, a hydroxyl group, an aldehyde group, an epoxy group and so on, which are present on the surface of the magnetic particle, to various types of functional groups on the receptor, and directly fixing the receptor on the magnetic particle, a method of chemically bonding the magnetic particle and the receptor with a spacer molecule interposed therebetween, and a method of chemically bonding the receptor to another protein such as albumin and then chemically bonding the protein to the magnetic particle. The chemically bonding method may use all the conventional methods.

Next, a process of bonding the first receptor to which the first ligand in the target nucleic acid specifically binds to the bottom of the container will be described.
The container as used herein may be a transparent, semi-transparent, or colored container. The material of the container may be plastic such as polystyrene, polypropylene, Teflon (registered trademark), polyethylene, methylpentene resin (TPX), fluorocarbon resin, acrylic resin, polycarbonate, polyurethane, vinyl chloride resin or the like. The container may be molded by the conventional method such as molding.
As the method of bonding the first receptor to which the first ligand in the target nucleic acid specifically binds to the bottom of the container, a method of bonding the receptor to the particle or the magnetic particle may be used and the method may be properly selected according to the kinds of the receptor bonded.

The receptor bonded to the magnetic particle or the container includes, but not limited to, for example, an antibody, lectin, streptavidin, nickel or the like may be used. The reason why nickel is available is because the nickel has chelate forming ability.

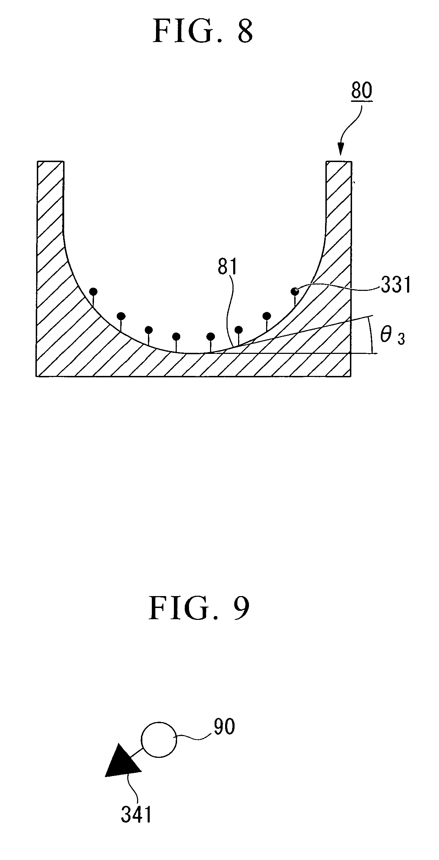

In the present method, since at least two kinds of ligands are bonded to the target nucleic acid, at least two kinds of receptors are prepared with respect to one kind of target nucleic acid. In addition, the container 80 (FIG. 8) in which the first receptor 331 to which the first ligand 31 specifically binds is bonded to the bottom and the magnetic particle 90 (FIG. 9) bonded with the second receptor 341 to which the second ligand 32 specifically binds are manufactured.

In the present invention, two or more kinds of receptors may be bonded to one magnetic particle and container. In the present embodiment, among two kinds or more ligands in one target nucleic acid, the receptor to which one kind of ligand specifically binds and the receptor excluding the receptors to which the remaining kind of ligands specifically bind are bonded to one magnetic particle or container. That is, only one kind of two or more kinds of ligands in the target nucleic acid is designed to be boned to one magnetic particle or container.

### (Process of Adding Sample which may have Target Nucleic Acid and Magnetic Particle in Container)

Next, a process of adding the sample which may have the target nucleic acid and the magnetic particle will be described.
In the present process, although a mixture may be added to the container after the sample having the possibility of including the target nucleic acid and the magnetic particle is mixed or the sample having the possibility of including the target nucleic acid and the magnetic particle may be simultaneously added to the container, the magnetic particle is preferably added to the container after the sample having the possibility of including the target nucleic acid is added to the container. The sample having the possibility of including the target nucleic acid and the magnetic particle are preferably used as solutions. In this case, a buffer solution is preferably used. As a preferable buffer solution, for example, PBS may be used and the buffer solution may be properly selected depending on the situation.

If the amount of the magnetic particle provided to addition to the container is too large, detection precision deteriorates. Thus, the amount of the magnetic particle is preferably slightly more than that of the target nucleic acid and is more preferably, specifically, 0.01 to 0.5 w/v% with respect to the target nucleic acid of several tens to several hundreds of nM. The number of first receptor bonded to the bottom of the container is larger than the number of first ligands in the target nucleic acid and is about 1.2 to 2 times of the number of ligands.

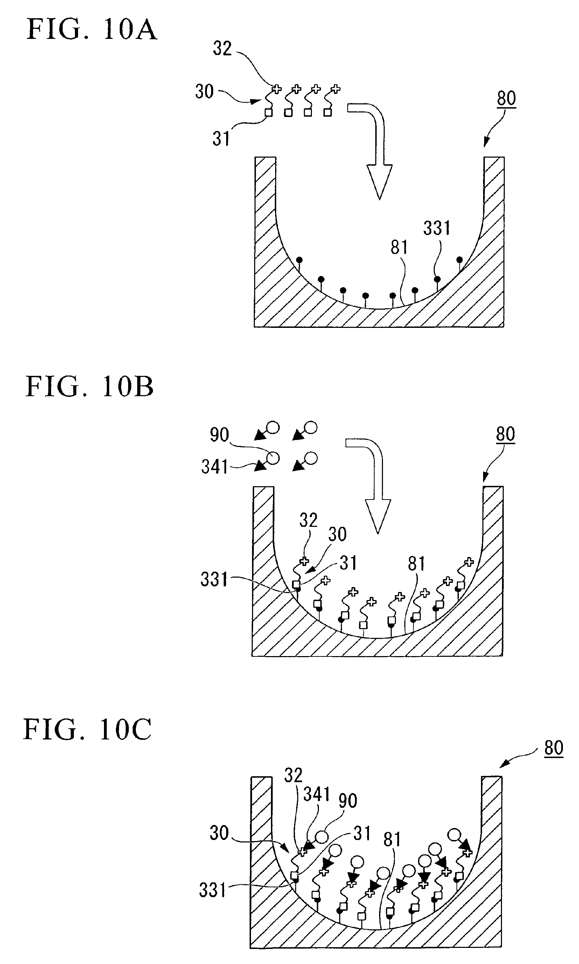

If the sample which may have the target nucleic acid and subjected to detection actually includes the target nucleic acid 30 shown in FIG. 10A, as shown in FIG. 10B, a target nucleic acid 30 is bonded to a bottom 81 of a container 80 by the bonding between the first ligand 31 and the first receptor 331, and, as shown in FIG. 10C, the target nucleic acid 30 and the magnetic particle 90 are bonded by the bonding between the second ligand 32 and the second receptor 341. That is, the container 80 and the magnetic particle 90 are bonded to each other with the target nucleic acid 30. If the sample subjected to detection does not include the target nucleic acid, this bonding does not occur and the magnetic particle 90 is not bonded to the container 80.

### (Process of Observing Distribution of Magnetic Particle on Bottom of Container)

Subsequently, the magnetic particle distribution pattern is observed using the method such as naked eyes or image processing when magnetic force is applied to the magnetic particle in the container by bringing a magnetic body such as a magnet into contact with the bottom of the container at the outside of the container. The image processing or the like uses the above-described method.

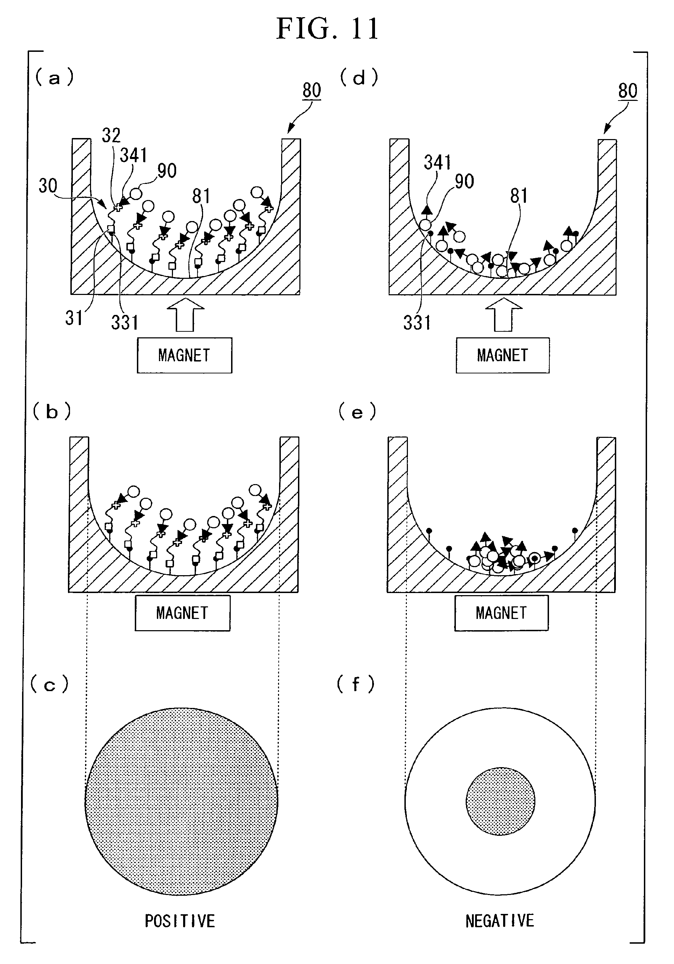

When the target nucleic acid is included in the sample (positive), as shown in FIG. 11 (panels (a) to (c)), although the magnetic particles 90 are tried to be collected by the magnetic body, the magnetic particles 90 cannot be collected because the container 80 and the magnetic particle 90 are bonded with the target nucleic acid 30. Thus, the magnetic particles 90 are held over substantially the entire surface of the bottom 81 of the container 80. If the amount of target nucleic acid 30 is large, the number of magnetic particles 90 for covering the bottom 81 of the container 80 is increased and, if the amount of target nucleic acid 30 is small, the number of the magnetic particles 90 for covering the bottom 81 is decreased. Accordingly, the color density of the bottom 81 varies according to the concentration of the target nucleic acid 30 in the sample. In contrast, when the target nucleic acid is not included in the sample (negativity), as shown in FIG. 11 (panels (d) to (f)), the magnetic particles 90 are collected in the vicinity of the magnetic body of the bottom 81 by the magnetic body.
Since the magnetic particle distribution pattern .on the bottom of the container varies depending on the presence/absence or a difference in the concentration of the target nucleic acid in the sample subjected to measurement, it is possible to easily detect the target nucleic acid with high precision. The panels (a) and (b) in FIG. 11 are longitudinal cross-sectional views of the container 80 in a state in which the magnetic particle 90 is bonded to the container 80, The panels (d) and (e) in FIG. 11 are longitudinal cross-sectional views of the container 80 in a state in which the magnetic particle 90 is not bonded to the container 80, and FIGS. 11C and 11F are views the bottom of the container 80 when viewed from the top.

In the case where, for example, an antigen and an antibody are used as a combination of the ligand and the receptor, the present process is preferably performed at 0 to 37 °C for 5 to 30 minutes. Even in the other combination of the ligand and the receptor, a suitable temperature, time and buffer solution may be properly selected according to the kinds of the combination.

Next, the detailed method of detecting the target nucleic acid will be described.
The target nucleic acid is added into the container by the above-described method using a sample (1) including the target nucleic acid having the known concentration and bonded with the ligand, the magnetic particle is added into the container, and the magnetic particle distribution pattern when the magnetic force is applied is checked and is set to be a positive control. In addition, a sample (2) including no target nucleic acid is added into the container, the magnetic particle is added into the container, and the magnetic particle distribution pattern when the magnetic force is applied is checked and is set to be a negative control. In addition, a sample (3) which may have target nucleic acid is added into the container, the magnetic particle is added into the container, and the magnetic particle distribution pattern on the bottom of the container is compared with the positive control and the negative control so as to check the presence/absence or the concentration of the target nucleic acid in the sample (3).

The magnetic particle distribution pattern on the bottom of the container, which is obtained using the sample including the target nucleic acid having the known different concentration, is set to be a control and the magnetic particle distribution when a sample (4) having a possibility of including the target nucleic acid is compared with the control so as to check the presence/absence or the concentration of the target nucleic acid in the sample (4).
In addition, when a difference in the magnetic particle distribution due to a difference in the presence/absence or the concentration of the target nucleic acid is checked and recorded in advance, it is possible to check the presence/absence or the concentration of the target nucleic acid in the sample by only subjecting the sample which may have the target nucleic acid to the detection without using the control. Accordingly, it is possible to simplify the detecting process.

In addition, in the case where plural kinds of target nucleic acids are included in the sample, for example, it is possible to selectively detect one kind of target nucleic acid, by utilizing different first and second ligands according to the kinds of the target nucleic acid and adding only the magnetic particle bonded with the second receptor to which the second ligand in one kind of target nucleic acid specifically binds into the container bonded with the first receptor to which the first ligand in one kind of target nucleic acid among plural kinds of target nucleic acids specifically binds. Similarly, it is possible to simultaneously detect two or more kinds of target nucleic acids, by adding all the magnetic particles bonded with the second receptor to which the second ligand in plural kinds of target nucleic acids specifically binds into the container bonded with the first receptor to which the first ligand in plural kinds of target nucleic acids specifically binds.
Accordingly, it is possible to solely detect the nucleic acid of the detection object or simultaneously detect nucleic acids from the plurality of the detection objects.

In the first and second ligands in the target nucleic acid, although the different ligands may be used according to the kinds of the target nucleic acid as described above, one of the first and second ligands may be commonly used in the plural kinds of nucleic acids. Accordingly, it is possible to simplify the detecting process.

Although, in the above-described detecting method, two kinds of ligands are used with respect to one kind of target nucleic acid, three or more kinds of ligands may be used. In this case, although the receptor to which each ligand specifically binds is used, among three kinds of receptors, the receptor bonded to the container and the receptor bonded to the magnetic particle are properly selected according to their purpose.

### (Container Used for Detecting Target Nucleic Acid)

As the container used in the present process, for example, a container shown in FIG. 8 is used, in consideration of the facilitation of the magnetic particles which are not bonded to the container when the magnetic force is applied to the magnetic particle. That is, the bottom 81 has a substantially semi-spherical shape and an angle (θ₃) between the bottom 81 and the horizontal plane is preferably 15 to 45 °. The bottom 81 may have a substantially conical surface shape instead of the substantial semi-spherical shape. In more detail, the angle (θ₃) indicates an angle between the horizontal plane and a straight line connected between any one of an inclined surface of the bottom 81 and the center of the bottom 81.
Similar to the first method, the container may be solely used or a plurality of containers provided on the same plate may be used. In more detail, for example, a 96-well microplate may be used as the container. The number of wells is not limited to 96. In the case where the plurality of containers is used, the capacity of one container is preferably 0.1 to 0.5 ml. The container used in the second method is obtained by bonding the receptor to the bottom of the container molded by the conventional method such as molding.

With respect to the portion excluding the above-described portion of the second method, the same method as the first method is applicable to the second method.

In the present invention, by utilizing the first method and the second method together, it is possible to detect the target nucleic acid with higher precision. In addition, in the first method, as described above, by utilizing the containers described in FIG. 4 and FIG. 5 together, it is possible to detect the target nucleic acid with even higher precision.

As described above in detail, according to the method of detecting the target nucleic acid of the present invention, it is possible to easily detect the nucleic acid of the detection object with low cost and high precision. Accordingly, for example, it is possible to realize low cost and facilitation of a clinical examination.

The method of detecting the target nucleic acid of the present invention may be, for example, used as means for detecting single nucleotide polymorphism (SNP) of the nucleic acid of the detection object. In this case, in the case where the PCR method is used as the method of obtaining the target nucleic acid boded with a plurality of different haptens, for example, a Polymerase Chain Reaction-Sequence Specific Primer (PCR-SSP) method may be suitably used. The PCR-SSP method indicates a method of designing a sequence such that 3'- terminal of one of two kinds of primers overlap with an SNP site in the nucleic acid of the detection object so as to control the amplification due to the PCR by the presence/absence of matching with the 3'- terminal of the primer and the SNP site.

Hereinafter, a principle will be described in detail.
As a primer bonded with a first ligand, for example, a primer in which a digoxigenin (DIG) is bonded to 5'- terminal of the primer including a base sequence represented by SEQ ID No. 2, a primer in which an FITC is bonded to 5'- terminal of the primer including a base sequence represented by SEQ ID No. 3, and a primer in which a biotin is bonded to 5'- terminal of the primer including a base sequence represented by SEQ ID No. 4 are manufactured. Next, with respect to the nucleic acid of the detection object including a base sequence represented by SEQ ID No. 5, an amplification reaction is performed by the PCR-SSP method. At this time, as described by r in the base sequence represented by SEQ ID No. 5, in the SNP site of the sample, when two types including a base A (adenine) and a base G (guanine) are present as aryl, the aryl type becomes three types including homo of A/A, hetero of A/G and homo of G/G.
If the SNP site of the sample is the homo of A/A, the DIG-labeled primer including SEQ ID No. 2 is matched with the SNP site and the amplification of the nucleic acid of the detection object is performed by the PCR together with the biotin-labeled primer including SEQ ID NO. 4. Since the amplification is not performed in the FITC-labeled primer including SEQ ID No. 2, an amplification product becomes a double-stranded DNA (target nucleic acid 1) in which the DIG and the biotin are separately bonded to both 5'- terminals.
Similarly, if the SNP site of the sample is the homo of G/G, a PCR product becomes a double-stranded DNA (target nucleic acid 2) in which the FITC and the biotin are separately bonded to both 5'- terminals.
If the SNP site of the sample is the hetero of A/G, it is possible to obtain an amplification product in which the target nucleic acid 1 and the target nucleic acid 2 are mixed. By detecting the presence/absence of the amplification of the nucleic acid, SNP typing becomes possible.
If the SNP of the nucleic acid is detected by the above-described principle, it is possible to obtain the target nucleic acid modified by the hapten corresponding to the polymorphism.
In addition, it is possible to detect a plurality of SNPs of the nucleic acid of the detection object. In this case, primers bonded with different kinds of haptens are prepared whenever the SNP site is changed and the nucleic acid of the detection object is amplified by the PCR-SSP method according to the above-described method.

Hereinafter, the present invention will be described in more detail by the detailed examples. However, the present invention is not limited to the following examples.
Now, examples of the SNP typing based on the above-described principle are described.

### [Example 1]

### <Extraction of DNA (Nucleic Acid of Detection Object)>

A DNA was extracted from a human blood. In more detail, an automatic nucleic acid extractor "Magtration System 6 CG (manufactured by Precision System Science Co., Ltd.)" was used and the protocol was followed by the instruction handbook of the same product.
(1) A subject's blood was drawn by 5 ml.
(2) 100 µl of the drawn blood was dispensed to a 1.5-ml screw cap type tube.
(3) A device was started up, an extracting reagent and a subject were set, and DNA extraction was started up by manipulating a machine according to a manipulation program. As an extraction liquid, TE (10 mM Tris - HCl(pH8.0 or 7.5) + 1mM EDTA) was used.
(4) After extraction, an absorbance was measured using a spectral photometer (manufactured by Nano Drop Technologies Inc. of U.S.) and a concentration after extraction was measured.
   By the above method, the bloods of nine subjects with known aryl types of the target SNP were drawn in advance, the DNAs thereof are extracted, and the genomic DNAs having the concentrations shown in Table 1 were obtained by 100 µl from the 100µl of total blood.

**[Table 1]**

| Subject No. | 1 | 2 | 3 | 4 | 5 | 6 | 7 | 8 | 9 |
|---|---|---|---|---|---|---|---|---|---|
| Aryl type | A/A | A/A | A/A | A/G | A/G | A/G | G/G | G/G | G/G |
| DNA concentration (ng/µl) | 1.82 | 1.96 | 1.83 | 1.71 | 1.69 | 1.92 | 1.88 | 1.83 | 1.85 |

### <Amplification of Nucleic Acid of Detection Object Bonded with Ligand>

Using a primer in which a DIG is bonded to 5'- terminal of the primer including a base sequence represented by SEQ ID No. 2, a primer in which an FITC is bonded to 5'- terminal of the primer including a base sequence represented by SEQ ID No. 3, and a primer in which a biotin is bonded to 5'- terminal of the primer including a base sequence represented by SEQ ID No. 4, the amplification of the nucleic acid of the detection object having the base sequence represented by SEQ ID No. 5 including the SNP was performed by the PCR using the genomic DNA as a template.
(1) The concentration of the genomic DNAs was adjusted with the TE so as to become 20 ng/µl.
(2) The reagents of the subjects were prepared as shown in Table 2.
(3) The prepared reagents were injected to PCR tubes in 20 µl.
(4) The subject's solutions were added into the PCR tubes of (3) in 1 µl.
(5) The PCR tubes were set in a thermal cycler "Gene AmpPCRsystem9700 (manufactured by Applied Biosystems Inc.) and the PCR reaction was performed under the condition shown in Table 3.

**[Table 2]**

| Amount of reagent per subject | |
|---|---|
| 10×PCR buffer solution | 2µl |
| dNTP mix | 1.6µl |
| DIG-labeled forward primer | 1µl |
| FITC-labeled forward primer | 1µl |
| Biotin-labeled reverse primer | 1µl |
| dH₂O | 13.3µl |
| ExTaq | 0.1µl |
| Sum | 20µl |

**[Table 3]**

| | | |
|---|---|---|
| 1 | 94°C | 5 minutes |
| 2 | 94°C | 30 seconds |
| 3 | 60°C | 30 seconds |
| 4 | 72°C | 30 seconds |
| 5 | 72°C | 5 minutes 2∼4 × 35 |

By the above method, the amplification reaction of the target nucleic acid was performed with the subjects 1 to 9.
A double-stranded artificial synthetic target nucleic acid (I) including a primer
in which a DIG is bonded to 5'- terminal of the primer including a base sequence represented by SEQ ID No. 6 and a primer in which a biotin is bonded to 5'- terminal of the primer including a base sequence represented by SEQ ID No. 7 was prepared, a double-stranded artificial synthetic target nucleic acid (II) including a primer in which an FITC is bonded to 5'- terminal of the primer including a base sequence represented by SEQ ID No. 8 and a primer in which a biotin is bonded to 5'- terminal of the primer including a base sequence represented by SEQ ID No. 9 was prepared, the artificial synthetic target nucleic acids (I) and (II) were mixed, the concentrations thereof were adjusted with the TE such that the artificial synthetic target nucleic acids (I) and (II) become 100 nM, used as a positive control. In addition, the TE was used as a negative control.

### <Detection of Target Nucleic Acid>

The target nucleic acid bonded with the ligand, which was manufactured in the above process, was detected.
Hereinafter, the procedures of the detecting method and the detected result will be described.
The target nucleic acid was detected using the container shown in FIG. 4.
(1) A solution of a latex particle A (diameter: 0.1 µm, manufactured by Seradyn Inc.) sensitized with an anti-DIG antibody (manufactured by DAKO, catalog No: D5105) was diluted using a buffer solution (200 mM MOPSO (pH7.4), 4% dextran, 100 mM NaCl, 0.1% sodium azide) and was injected to a 96-well reaction plate in 50 µl per well using the container a (h: 0.2 µm, 1:0.5 µm, θ₁: 30°) shown in FIG. 4, which is manufactured by molding.
(2) A solution of a latex particle sensitized with an anti-biotin antibody (manufactured by DAKO, catalog No: M0743) was diluted using the above-described buffer solution and was injected into the 96-well plate by 50 µl per well.
(3) The subject, the positive control and the negative control were added in 20 µl and were dispersed well.
(4) After being left at a room temperature for 5 minutes, the positive control and the negative control were compared, and the sediment capturing distribution of the subject on the bottom of the container was observed so as to be determined to positivity or negativity.

### [Example 2]

Except that a solution of a latex particle B (diameter: 10 µm, manufactured by Magsphere Inc.) sensitized with an anti-FITC antibody (manufactured by DAKO, catalog No: D5101) was used instead of the solution of the latex particle A sensitized with the anti-DIG antibody and a 96-well reaction plate using the container b (h: 20 µm, 1: 50 µm, θ₁: 30°) shown in FIG. 4, which is manufactured by molding, as the well was used instead of the container a, the target nucleic acid was detected by the same method as Example 1.

### [Example 3]

The target nucleic acid was detected using the container shown in FIG. 5.
Except that a 96-well reaction plate using a container c (L: 0.05 µm, d: 0.03 µm, θ₂: 30°) shown in FIG. 5, which is manufactured by molding, as the well is used, instead of the container a, the target nucleic acid was detected by the same method as Example 1.

### [Example 4]

Except that a solution of a latex particle B sensitized with an anti-FITC antibody (manufactured by DAKO, catalog No: D5101) was used instead of the solution of the latex particle A sensitized with the anti-DIG antibody and a 96-well reaction plate using the container d (L: 100 µm, d: 60 µm, θ₂: 30°) shown in FIG. 5, which is manufactured by molding, as the well was used instead of the container c, the target nucleic acid was detected by the same method as Example 3.

### [Example 5]

The target nucleic acid was detected using the container shown in FIG. 8.
(1) An anti-biotin antibody was bonded to the bottom of a 96-well plate [(immobilizer amino Rockwell module plate (manufactured by Nunc, catalog No: 436023)) according to the known method.
(2) The above-described buffer solution was dividedly injected by 50 µl per well.
(3) The subject, the positive control and the negative control were dividedly injected in 20 µl.
(4) It was left at a room temperature for 5 minute.
(5) A solution of a magnetic particle [Dynabeads M - 450 Epoxy (manufactured by DYNAL, catalog No: DB14001)] bonded with an anti-DIG antibody was divided by 50 µl per well and was dispersed well.
(6) It was left at a room temperature for 5 minute.
(7) A magnet was brought into contact to the outside of the bottom of the plate, the positive control and the negative control were compared, the magnetic particle distribution pattern on the bottom of the container was observed by the method such as naked eyes or image processing so as to be determined to positivity or negativity.

### [Example 6]

Except that a magnetic particle bonded with an anti-FITC antibody was used instead of the magnetic particle bonded with the anti-DIG antibody, the target nucleic acid was detected by the same method as Example 5.

### (Determined Result)

The detection results of Examples 1 to 6 and the overall decision based on the detection result are shown in Table 4. As the overall decision, if at least three detected results are equal among the determination results of Examples 1 to 6, the result was the final typing result. It can be seen that it is possible to detect the target nucleic acid with high precision by the present invention.

**[Table 4]**

| SubjectNo. aryl type | 1 | | 2 | | 3 | | 4 | | 5 | | 6 | | 7 | | 8 | | 9 | |
|---|---|---|---|---|---|---|---|---|---|---|---|---|---|---|---|---|---|---|
| | AA | | AA | | AA | | AG | | AG | | AG | | GG | | GG | | GG | |
| Detected aryl | A | G | A | G | A | G | A | G | A | G | A | G | A | G | A | G | A | G |
| Example 1 | + | - | + | - | + | - | + | + | + | + | + | + | - | + | - | + | - | +/- |
| Example 2 | + | - | + | - | + | - | + | + | + | + | + | + | - | +/- | - | + | - | + |
| Example 3 | + | +/- | +/- | - | + | - | + | + | + | + | +/- | + | +/- | + | - | + | - | + |
| Example 4 | + | +/- | + | - | + | - | + | + | +/- | + | + | + | +/- | + | - | + | - | + |
| Example 5 | + | - | + | +/- | + | - | +/- | + | + | + | + | + | - | + | - | + | +/- | + |
| Example 6 | + | - | + | - | + | - | + | + | + | + | + | + | - | + | - | + | - | + |
| Overall Decision | + | - | + | - | + | - | + | + | + | + | + | + | - | + | - | + | - | + |
| Typing result | A/A | | A/A | | A/A | | A/G | | A/G | | A/G | | G/G | | G/G | | G/G | |

| | | | | | | | | | | | | | | | | | | |
|---|---|---|---|---|---|---|---|---|---|---|---|---|---|---|---|---|---|---|
| +: positivity, -: negativity, +/-: negativity and positivity are hard to be distinguished | | | | | | | | | | | | | | | | | | |

### Industrial Applicability

The present invention is suitable for facilitation and low cost of a clinical examination and is available in the medical field.

## Claims

1. A method of detecting a target nucleic acid, in which a first ligand and a second ligand, which are ligands of different kinds, are bonded to a nucleic acid of a detection object, the method comprising the steps of:
preparing a first particle bonded with a plurality of first receptors to which the first ligand specifically binds and a second particle bonded with a plurality of second receptors to which the second ligand specifically binds;
mixing a sample which may have the target nucleic acid, the first particle and the second particle in a container having a precipitation capturing part on a bottom thereof; and
observing a capturing distribution of agglomerates including the target nucleic acid with the first particle and the second particle where the particles are linked through the bonding between the first ligand and the first receptor and the bonding between the second ligand and the second receptor, respectively, and/or a precipitation of the first particle and the second particle, on the bottom of the container, after the mixing step.

2. A method of detecting a target nucleic acid, in which a first ligand and a second ligand, which are ligands of different kinds, are bonded to a nucleic acid of a detection object, the method comprising the steps of:
preparing a container in which a plurality of first receptors to which a first ligand specifically binds is bonded to a bottom thereof;
preparing a magnetic particle in which a plurality of second receptors to which a second ligand specifically binds is bonded;
adding a sample which may have the target nucleic acid and the magnetic particle into the container; and
applying a magnetic force from the outside of the container to the magnetic particle and observing a magnetic particle distribution on the bottom of the container, after the adding step.

3. A method of detecting a target nucleic acid, the method comprising performing the detecting method according to claim 1 and the detecting method according to claim 2.

4. The method according to any one of claims 1 to 3, wherein an observation result obtained when a sample (1) including a target nucleic acid having the known concentration is used, an observation result obtained when a sample (2) including no target nucleic acid is used, and an observation result obtained when a sample (3) which may have the target nucleic acid is used are compared so as to check the presence/absence or the concentration of the target nucleic acid in the sample (3).

5. The method according to any one of claims 1 to 3, wherein an observation result obtained when a sample including target nucleic acids having the known different concentrations are used and an observation result obtained when a sample (4) which may have the target nucleic acid is used are compared so as to check the presence/absence or the concentration of the target nucleic acid in the sample (4).

6. The method according to any one of claims 1 to 5, wherein the ligand is selected from the group consisting of a hydrophilic organic compound, digoxigenin, fluorescein, alexa, fluorescein isothiocyanate (FITC), 2,4-dinitrophenol (DNP), tetramethyl rhodamine (TAMRA), a polypeptide of 6 or more amino acid residues, a sugar chain having two or more monosaccharides, a biotin, a protein, a polyhistidine, HA, a glutathione S-transferase (GST), a peptide (Flag) including an amino acid sequence represented by SEQ ID No. 1, and derivatives of any of these.

7. The method according to any one of claims 1 to 6, wherein the target nucleic acid is one prepared by a polymerase chain reaction method using a first primer to which the first ligand is bonded and a second primer to which the second ligand is bonded.

8. The method according to any one of claims 1 to 6, wherein the target nucleic acid is prepared by a polymerase chain reaction method while at least one nucleotide to which the first ligand is bonded is introduced using a first primer; and at least one nucleotide to which the second ligand is bonded is introduced using the second primer.

9. The method according to any one of claims 1 to 8, wherein plural kinds of different target nucleic acids are used, where at least one of the first ligand and the second ligand in the target nucleic acids is a different kind for each of the target nucleic acids.

10. The method according to any one of claims 1 to 9, wherein the target nucleic acid is derived from an organism.

11. The method according to any one of claims 1 and 3 to 10, wherein the first and second particles have a diameter of 0.1 to 10 µm and are optically distinguishable from each other.

12. The method according to any one of claims 1 to 11, wherein a plurality of containers is provided on the same plate and the capacity of each of the containers is 0.1 to 0.5 ml.

13. The method according to any one of claims 1 and 3 to 12, wherein, the precipitation capturing part of the container is formed on the bottom of thereof having a conical shape, so that a plurality of peak-valley forms consisting of peak portions and valley portions which are alternatingly arranged, and having a diameter-direction cross section of a saw-tooth shape, is concentrically arranged; the length (h) of a first inclined surface connecting a peak and a valley from a center to the outside of the bottom is 0.2 to 20 µm, the length (1) of a second inclined surface connecting a valley and a peak from the center to the outside of the bottom is 0.5 to 50 µm, and an angle (θ₁) between a straight line connected between the peak and the center, and a horizontal plane is 15 to 45°.

14. The method according to any one of claims 1 and 3 to 12, wherein, in the precipitation capturing part of the container, a plurality of valley parts is independently provided on the bottom having a conical shape and an angle (θ₂) between the bottom and a horizontal plane is 15 to 45°.

15. The method according to claim 14, wherein the diameter (L) of an opening of each of the valley parts is 0.05 to 100 µm and the depth (d) thereof is 0.03 to 60 µm.

16. A method of detecting a target nucleic acid, the method comprising performing the detecting method according to claim 13 and the detecting method according to claim 14 or 15.

17. The method according to any one of claims 2 to 10 and 12, wherein the diameter of the magnetic particle is 0.1 to 20 µm.

18. The method according to any one of claims 2 to 10, 12 and 17, wherein the bottom of the container has a substantially semi-spherical shape or a substantially conical surface shape and an angle (θ₃) between the bottom and a horizontal plane is 15 to 45°.

19. A container used for the method of detecting the target nucleic acid according to any one of claims 1 and 3 to 12, wherein, the precipitation capturing part of the container is formed on the bottom of thereof having a conical shape, so that a plurality of peak-valley forms consisting of peak portions and valley portions which are alternatingly arranged, and having a diameter-direction cross section of a saw-tooth shape, is concentrically arranged; the length (h) of a first inclined surface connecting a peak and a valley from a center to the outside of the bottom is 0.2 to 20 µm, the length (1) of a second inclined surface connecting a valley and a peak from the center to the outside of the bottom is 0.5 to 50 µm, and an angle (θ₁) between a straight line connected between the peak and the center and a horizontal plane is 15 to 45°.

20. A container used for the method of detecting the target nucleic acid according to any one of claims 1 and 3 to 12, wherein a plurality of valley parts is independently provided on the bottom having a conical shape and an angle (θ₂) between the bottom and a horizontal plane is 15 to 45°.

21. The container according to claim 20, wherein the diameter (L) of an opening of each of the valley parts is 0.05 to 100 µm and the depth (d) thereof is 0.03 to 60 µm.

22. A container used for the method of detecting the target nucleic acid according to any one of claims 2 to 10, 12 and 17, wherein the bottom thereof has a substantially semi-spherical shape or a substantially conical surface shape and an angle (θ₃) between the bottom and a horizontal plane is 15 to 45°.

23. A container used for detecting a target nucleic acid, in which the container according to any one of claims 19 to 22 is plurally provided on the same plate.
